# EUROPEAN PATENT SPECIFICATION

(11) **EP 4 582 026 B1**
(45) Date of publication and mention of the grant of the patent: **04.11.2026**
(21) Application number: 25150086.4
(22) Date of filing: 02.01.2025
(51) Int. Cl.: A61B 6/04, A61B 5/00, A61B 6/10

(54) **CLUTCH RELEASE DEVICE AND MEDICAL TABLE**
KUPPLUNGSLÖSEVORRICHTUNG UND MEDIZINISCHER TISCH
DISPOSITIF DE LIBÉRATION D'EMBRAYAGE ET TABLE MÉDICALE

(30) Priority: 08.01.2024 CN 202420042195 U
(43) Date of publication of application: 09.07.2025
(73) Proprietor: Siemens Healthineers AG, 91301 Forchheim (DE)
(72) Inventor: LI, Qiu li, Shanghai, 201203 (CN); ZHU, Ping, Shanghai, 200120 (CN)
(74) Representative: HKW Intellectual Property PartG mbB

(56) References cited:
- EP-A1- 3 811 870
- CN-U- 216 812 585

## Description

### TECHNICAL FIELD

The present application relates to the technical field of machinery, and in particular to a clutch release device and a medical table.

### BACKGROUND ART

A CT/MR medical table is provided with a clutch device between a driving motor and a table board transmission device, which is used to ensure that the table can be moved safely in various situations and to reduce the risk of injury to patients. In an actual application of the CT/MR medical table, in the case of occurrence of a safety hazard to a patient or in other special circumstances, it is necessary to manually pull the clutch device to temporarily disengage the driving motor from the table board transmission device, so that the medical staff can manually move the table board, causing the patient to escape the safety hazard as quickly as possible. Then, the clutch is manually pulled to quickly engage the driving motor and the transmission device to resume the working state. The use of the clutch device can provide additional safety protection for patients and medical workers, and can reduce the occurrence of medical accidents to a certain extent.

CN 216812585 U provides a clutch device, a driving device and a scanning bed. The clutch device can be operated to decouple a transmission mechanism and a motor. The clutch device comprises a clutch part, and when the clutch part is set to be coupled with the transmission mechanism, the motor is coupled with the transmission mechanism through the clutch part. A shifting rod is arranged to be coupled with the clutch part so as to apply acting force to the clutch part. A guide part is arranged to be in contact with one end of a deflector rod. The guide part is provided with a first guide surface and a second guide surface. The deflector rod is arranged to move to be in contact with the first guide surface or the second guide surface under the action of external force, and when the deflector rod moves to be in contact with the guide part along the second guide surface, a moving track of the deflector rod in contact with the first guide surface is changed. When a lateral acting force for changing the relative position between the clutch part and the transmission mechanism is applied to the clutch part, the clutch part is triggered to act to be decoupled from the transmission mechanism, and the motor is decoupled from the transmission mechanism.

The operator's action direction of the clutch device in the existing CT/MR medical table is in a different direction from the movement direction of the clutch disc, which will affect the convenience and intuitiveness of the operation and limit the application scenarios.

### SUMMARY OF THE APPLICATION

In view of the above-mentioned shortcomings of the prior art, an object of the present application is to provide a new clutch release device and a medical table.

To achieve the above object and other related objects, the present application provides a clutch release device, comprising:
a push-pull rail;
a clutch pull handle mounted on the push-pull rail, the clutch pull handle being moveable between a first station and a second station along the push-pull rail;
a transmission wheel sleeved outside the transmission shaft located at one side of the push-pull rail and being rotatable about the transmission shaft, the transmission wheel receiving power and torque from a motor to rotate about the transmission shaft;
a clutch disc sleeved outside the transmission shaft and located on one side of the transmission wheel, the clutch disc rotating synchronously with the transmission shaft, and the clutch disc being movable between station a and station b along an axial direction of the transmission shaft; and
a connecting rod mechanism provided between the clutch disc and the clutch pull handle, the connecting rod mechanism being configured such that when the clutch pull handle is in the first station, the clutch disc is in station a, and the clutch disc is coupled to and rotates synchronously with the transmission wheel, and when the clutch pull handle is in the second station, the clutch disc is in station b, and the clutch disc is separated from the transmission wheel.

A working direction of the clutch pull handle is parallel to the axial direction of the transmission shaft.

In an optional embodiment of the present application, the clutch release device further comprises a locking component provided between the clutch pull handle and the push-pull rail, the locking component being used to achieve locking when the push-pull rail is in the first station or the second station.

In an optional embodiment of the present application, the locking component comprises a buckle and a slot that cooperate with each other, the buckle is suspended on one of the clutch pull handle and the push-pull rail, and the slot is provided on the other of the clutch pull handle and the push-pull rail.

In an optional embodiment of the present application, the buckle is connected to the clutch pull handle or the push-pull rail by an arc-shaped transition section.

In an optional embodiment of the present application, the clutch release device comprises two push-pull rails provided in parallel, which are provided on both sides of the clutch pull handle, respectively.

In an optional embodiment of the present application, at least two guide portions are spaced apart on both sides of the clutch pull handle, respectively, and the guide portions are located between the clutch pull handle and the push-pull rail on the same side.

In an optional embodiment of the present application, the connecting rod mechanism comprises a clutch lever and a rotating connecting rod;
the rotating connecting rod comprises a first fulcrum and a first segment and a second segment located on both sides of the fulcrum, the first fulcrum is rotatably mounted at a first fixed position, the first segment is connected to the clutch pull handle through a first guide pin provided on the clutch pull handle, the first guide pin can slide along a length direction of the first segment, the second segment is connected to the clutch lever through a second guide pin provided on a first end of the clutch lever, the second guide pin can slide along a length direction of the second segment of the rotating connecting rod;
a second fulcrum of the clutch lever is fixed at a second fixed position, and the positions of the first fixed position and the second fixed position remain relatively unchanged; and
when the clutch pull handle moves from the first station to the second station, the first guide pin slides along the length direction of the first segment in a direction away from the first fulcrum, driving the rotating connecting rod to rotate, thereby driving the second guide pin to slide along the length direction of the second segment in the direction away from the first fulcrum, and further driving the clutch lever to rotate about the second fulcrum, driving the clutch disc to move in a direction away from the transmission wheel, and moving the clutch disc from station a to station b.

In an optional embodiment of the present application, a distance between the first end of the clutch lever and the second fulcrum is greater than a distance between a second end and the second fulcrum.

In an optional embodiment of the present application, the clutch release device further comprises a return spring, the return spring being used to return the clutch disc to station a after the clutch disc is released.

In an optional embodiment of the present application, the clutch release device further comprises a spring stopper, wherein the spring stopper is fixedly mounted on the transmission shaft and is located on a side of the clutch disc away from the transmission wheel; and
the return spring is provided between the spring stopper and the clutch disc, one end of the return spring abuts against the clutch disc, and the other end abuts against the spring stopper.

In an optional embodiment of the present application, the return spring is sleeved on the transmission shaft.

In an optional embodiment of the present application, a first meshing component is provided on an end face of the clutch disc opposite to the transmission wheel, and a second meshing component is provided on an end face of the transmission wheel opposite to the clutch disc; and when the clutch disc moves between station a and station b along the axial direction of the transmission shaft, the first meshing component and the second meshing component form a clutch structure to couple and separate the clutch disc and the transmission wheel.

In an optional embodiment of the present application, the first meshing component and the second meshing component are designed with helical teeth, and an included angle between a side of a helical tooth and an axial direction of the helical tooth is between 5° and 15°.

In an optional embodiment of the present application, the included angle between the side of the helical tooth and the axial direction of the helical tooth is between 7° and 10°.

In an optional embodiment of the present application, the clutch release device further comprises a self-resetting mechanism, comprising a first resetting component and a second resetting component having ramp surfaces;
wherein the ramp surfaces of the first resetting component and the second resetting component are placed such that the first resetting component is fixed to the clutch pull handle, and the second resetting component is fixedly connected to an external device; and
in a resetting process of the external device, the second resetting component is driven to move, and the movement of the second resetting component triggers the movement of the first resetting component, thereby driving the clutch pull handle to move to return to the first station.

To achieve the above object and other related objects, the present application provides a medical table, comprising:
a table body;
a table board provided on the table body and being movable relative to the table body;
a table board transmission device provided on the table body, the table board transmission device being connected to the table board and being used to drive the table board to move, and the table board transmission device having a transmission shaft;
a motor provided on the table body; and
a clutch release device as disclosed above, the clutch release device being provided between the motor and the table board transmission device, and being used to control a transmission state between the table board transmission device and the motor;
wherein the push-pull rail is provided on the table body.

In embodiments, the clutch release device comprised in the medical table is implemented as disclosed above or below with respect to embodiments of the application and/or as claimed.

In an optional embodiment of the present application, a moving direction of the table board and a working direction of the clutch pull handle are perpendicular to each other.

The clutch release device of the present application comprises:
a fixed connecting plate; a push-pull rail provided on the fixed connecting plate;
a clutch pull handle mounted on the push-pull rail, the clutch pull handle being movable between a first station and a second station along the push-pull rail;
a transmission wheel sleeved outside the transmission shaft located on one side of the fixed connecting plate and being rotatable about the transmission shaft, the transmission wheel receiving power and torque from the motor to rotate about the transmission shaft; a clutch disc sleeved outside the transmission shaft and being located on one side of the transmission wheel, the clutch disc rotating synchronously with the transmission shaft, and the clutch disc being movable between station a and station b along an axial direction of the transmission shaft;
and a connecting rod mechanism provided between the clutch disc and the clutch pull handle, the connecting rod structure being configured such that when the clutch pull handle is in the first station, the clutch disc is in station a, and the clutch disc is coupled to and rotates synchronously with the transmission wheel, and when the clutch pull handle is in the second station, the clutch disc is in station b, and the clutch disc is separated from the transmission wheel.

The clutch release device of the present application can be used in a medical table to implement the transmission state control between the motor and the table board in the medical table, which can ensure that the table board of the medical table can be safely moved in various situations, and reduce the risk of injury to patients.

In the clutch release device of the present application, the working direction of the clutch handle is set to be parallel to the axial direction of the transmission shaft, so that the working direction of the clutch handle is consistent with the clutch direction, which is convenient for the operation of the clutch.

In the clutch release device of the present application, helical tooth meshing is adopted between the clutch disc and the transmission wheel. Compared with the design of straight tooth meshing, it not only ensures stable and effective transmission, but also eliminates the transmission backlash, while avoiding the risk of the clutch disc not being able to be pulled out due to the straight tooth design.

The clutch lever of the clutch release device of the present application adopts a labor-saving lever structure. During the operation, only a small force is required to realize the clutch between the clutch disc and the transmission wheel.

In the clutch release device of the present application, a buckle and a slot are adopted to realize clutch locking, which is ingeniously designed, flexible and reliable.

### BRIEF DESCRIPTION OF THE DRAWINGS

FIG. 1 is a schematic structural diagram of a clutch release device in an exemplary embodiment of the present application in a coupled state.
FIG. 2 is a schematic structural diagram of the clutch release device in the exemplary embodiment of the present application in a separated state.
FIG. 3 is a schematic diagram of the assembly of a push-pull rail, a clutch pull handle, a rotating connecting rod, and a clutch lever in an exemplary embodiment of the present application.
FIG. 4 is an exploded view of a push-pull rail and a clutch pull handle in an exemplary embodiment of the present application.
FIG. 5 shows a front view of a clutch disc with a helical tooth design in an exemplary embodiment of the present application.
FIG. 6 shows a top view of the clutch disc with the helical tooth design in the exemplary embodiment of the present application.
FIG. 7 shows a schematic structural diagram of a clutch release device with a self-resetting function in an exemplary embodiment of the present application.
FIG. 8 shows a cross-sectional view along line A-A in FIG. 7.

### DETAILED DESCRIPTION OF EMBODIMENTS

Embodiments of the present application will be explained below by way of specific particular examples, and those skilled in the art may easily understand other advantages and effects of the present application from the contents disclosed in this description. The present application may further be implemented or applied by way of other different particular embodiments; various modifications or changes may also be made to various details in this description, on the basis of different viewpoints and applications, without departing from the scope of the present application as defined in the attached set of claims.

As used in the present application, "a" does not only mean "just this one"; it may also mean "more than one". As used herein, "first" and "second" etc. are merely used to differentiate between parts, not to indicate their order or degree of importance, etc. In addition, a noun or pronoun referring to a person in the present disclosure is not limited to a specific gender.

This embodiment provides a medical table. The medical table may be, for example, a CT medical table or an MR medical table, and includes a table body, a table board, a table board transmission device, a motor, and a clutch release device. The table board is provided on the table body and can move relative to the table body. The table board transmission device is provided on the table body, and the table board transmission device is connected to the table board and is used to drive the table board to move. The motor is provided on the table body. The clutch release device is provided between the motor and the table board transmission device, and is used to control the transmission state between the table board transmission device and the motor. Of course, the clutch release device of this embodiment may also be applied to other scenarios where transmission clutch control is required, and is not limited to medical tables.

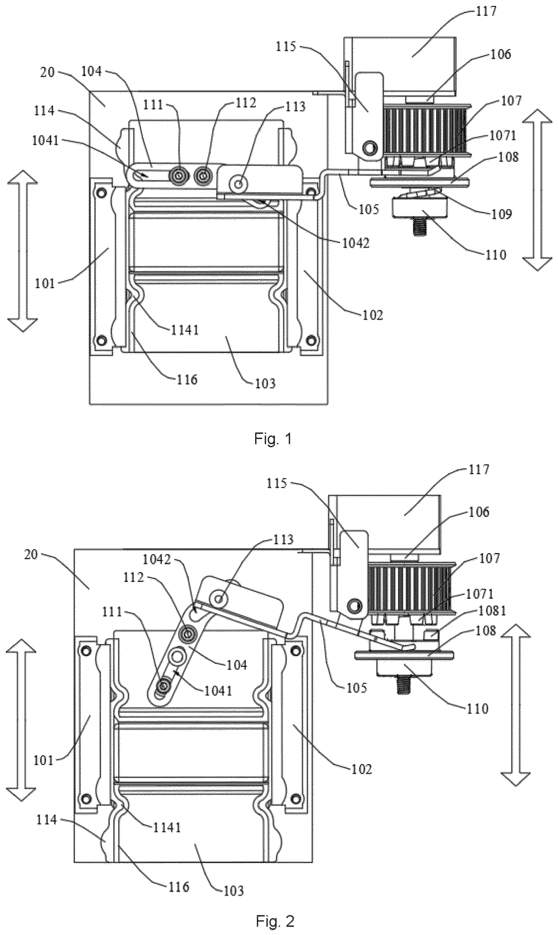

FIGS. 1 and 2 are exemplary structural diagrams of the clutch release device in a coupled state and a separated state, respectively. The clutch release device is used to control the transmission state between the table board transmission device 117 and the motor. The table board transmission device 117 has a transmission shaft 106 and a first transmission pulley (not shown) sleeved on the transmission shaft 106. The first transmission pulley is keyed to the transmission shaft 106, so that when the transmission shaft 106 rotates, the first transmission pulley can also rotate synchronously, thereby driving the table board to move. The clutch release device includes a push-pull rail, a clutch pull handle 103, a second transmission pulley 107 (as a transmission wheel), a clutch disc 108 and a connecting rod mechanism.

The push-pull rail is provided on the table body of the medical table. The clutch pull handle 103 is mounted on the push-pull rail, and the clutch pull handle 103 can move linearly between a first station shown in FIG. 1 and a second station shown in FIG. 2 along the push-pull rail. The second transmission pulley 107 is sleeved outside the transmission shaft 106 located on one side of the push-pull rail, and can rotate about the transmission shaft 106, and the second transmission pulley 107 receives the power and torque of the motor through a transmission belt to rotate about the transmission shaft 106. The clutch disc 108 is sleeved outside the transmission shaft 106, and is located on one side of the second transmission pulley 107. The clutch disc 108 rotates synchronously with the transmission shaft 106, and the clutch disc 108 can move between station a shown in FIG. 1 and station b shown in FIG. 2 along an axial direction of the transmission shaft 106. The connecting rod mechanism is provided between the clutch disc 108 and the clutch pull handle 103, and the connecting rod structure is configured such that when the clutch pull handle 103 is in the first station, the clutch disc 108 is in station a, and the clutch disc 108 is coupled to the second transmission pulley 107. When the second transmission pulley 107 rotates under the drive of the motor, it will drive the clutch disc 108 to rotate synchronously with the transmission shaft 106, and the rotation of the transmission shaft 106 can drive a first transmission pulley of the table board transmission device 117 to rotate, so as to drive the table board to move. When the clutch pull handle 103 is in the second station, the clutch disc 108 is in station b, and the clutch disc 108 is separated from the second transmission pulley 107, so that the transmission shaft 106 can rotate freely, and the table board can be moved freely at this time. The clutch release device of this embodiment can be used to implement the transmission state control between the motor and the table board in the medical table, which can ensure that the table board of the medical table can be safely moved in various situations, and reduce the risk of injury to patients.

A specific structure of the clutch release device of this embodiment will be described in detail below with reference to the drawings. For the convenience of description, with reference to the views in FIGS. 1 and 2, the up-down direction in FIGS. 1 and 2 is defined as the front-rear direction of the clutch release device, the left-right direction is defined as the left-right direction of the clutch release device, the inward direction perpendicular to the paper plane in FIGS. 1 and 2 is defined as down, and the outward direction perpendicular to the paper plane in FIGS. 1 and 2 is defined as up. In the subsequent description, the position and movement direction of each assembly will be explained according to the provided definitions.

Referring to FIGS. 1 and 2, in this embodiment, in order to mount the clutch release device, a fixed connecting plate 20 may be configured as a mounting base plate for mounting the push-pull rail, the clutch pull handle 103 and the connecting rod mechanism, and may be fixed on the table body of the medical table. The fixed connecting plate 20 is a plate-like structure, and an opening penetrating upper and lower surfaces is provided in the middle. The opening serves as a window for a handle portion 1034 of the clutch pull handle 103 to extend out. In a pulling process of the clutch pull handle 103, the handle portion 1034 can move forward and backward in the opening. It should be noted that, of course, the fixed connecting plate 20 may not be provided, and the push-pull rail, the clutch pull handle 103 and the connecting rod mechanism of the clutch release device may be directly mounted on the table body of the medical table.

Referring to FIGS. 1 and 2, in an optional embodiment, the clutch release device includes two push-pull rails spaced apart in parallel, which are a left push-pull rail 101 and a right push-pull rail 102, respectively. The left push-pull rail 101 and the right push-pull rail 102 are provided on the left and right sides of the clutch pull handle 103, respectively. By providing the left push-pull rail 101 and the right push-pull rail 102 on the left and right sides of the clutch pull handle 103, the stability and accuracy of the clutch pull handle 103 in motion can be ensured, so as to provide a good operating experience and a long service life.

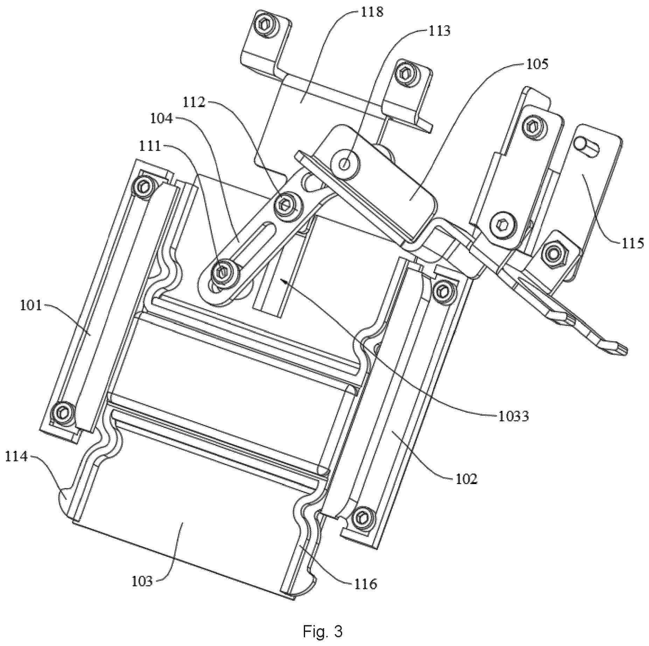

Referring to FIGS. 1-4, the clutch pull handle 103 is used as an operating component of the clutch release device. It is mounted on the fixed connecting plate 20 through the left push-pull rail 101 and the right push-pull rail 102, so that it can move back and forth linearly between the first station and the second station relative to the fixed connecting plate 20, that is, the working direction of the clutch pull handle 103 (the double-headed arrow on the left side in FIGS. 1 and 2) is the front-rear direction. A handle portion 1034 for an operator to push and pull the clutch pull handle 103 is protruded downward on one side of the clutch pull handle 103 facing the fixed connecting plate 20. The handle portion 1034 extends along the left-right direction of the fixed connecting plate 20 and extends from the opening of the fixed connecting plate 20. The handle portion 1034 can move back and forth in the opening of the fixed connecting plate 20.

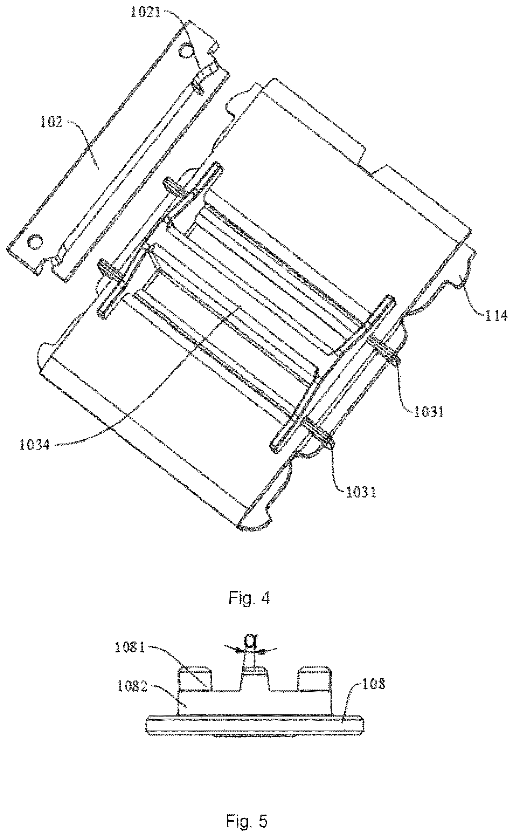

As shown in FIG. 4, further, two or more guide portions 1031 are spaced apart in the middle of the left and right sides of the clutch pull handle 103, and the guide portions 1031 are located between the clutch pull handle 103 and the push-pull rail on the same side. The guide portions 1031 can provide necessary support and positioning in the middle of the clutch pull handle 103 to ensure that the movement trajectory of the clutch pull handle 103 on the push-pull rail is stable and accurate. The design of the guide portions 1031 may be based on specific needs and design requirements, and may be protrusions, bulges, or other shapes to ensure that the movement of the clutch pull handle 103 can be effectively guided during the movement.

Referring to FIGS. 1 and 2, the second transmission pulley 107 is located on one side of the fixed connecting plate 20 and is sleeved on the transmission shaft 106 located on one side of the fixed connecting plate 20. The second transmission pulley 107 may rotate about the transmission shaft 106. The second transmission pulley 107 may be used as a passive transmission pulley, is connected to an active transmission pulley mounted on an output shaft of the motor through a transmission belt, and receives the power and torque of the motor to rotate about the transmission shaft 106, thereby transmitting the power and torque to the transmission shaft 106 of the table board transmission device through the coupled clutch disc 108 to drive the table board to move. It should be noted that in other embodiments, the second transmission pulley 107 may also be replaced with a gear, a sprocket, etc., and applied to driving scenarios such as gear transmission and chain transmission.

Referring to FIGS. 1, 2, 5 and 6, the clutch disc 108 is located on one side of the fixed connecting plate 20. The clutch disc 108 has a center hole 1083 for the transmission shaft 106 to pass through. The clutch disc 108 is sleeved on one end of the transmission shaft 106 located on the second transmission pulley 107 through the center hole 1083, and an inner wall of the center hole 1083 of the clutch disc 108 is connected to an outer wall of the transmission shaft 106 through a key and a keyway, so that the clutch disc 108 and the transmission shaft 106 can rotate synchronously with each other, while the clutch disc 108 can move between station a and station b along the axial direction of the transmission shaft 106. The movement direction of the clutch disc 108 (the double-headed arrow on the right side in FIGS. 1 and 2) is parallel to the axial direction of the transmission shaft 106, so that the working direction of the clutch pull handle 103 is consistent with the clutch direction, which is convenient for the clutch operation.

Referring to FIGS. 1 and 2, the clutch release device further includes a return spring 109 and a spring stopper 110. The return spring 109 is used to return the clutch disc 108 to station a after the clutch disc 108 is released. The spring stopper 110 may be fixedly mounted on the transmission shaft 106 by threading, and is located on the side of the clutch disc 108 away from the second transmission pulley 107. The return spring 109 is sleeved on the transmission shaft 106, and is located between the clutch disc 108 and the spring stopper 110. One end of the return spring 109 abuts against the clutch disc 108, and the other end abuts against the spring stopper 110. The function of the return spring 109 is to push the clutch disc 108 back to a starting position (i.e., station a) by elastic force after the clutch disc 108 is released by the clutch lever 105, so as to prepare for a next clutch action. The return spring 109 helps ensure that the clutch disc 108 can return to the starting position after each release, ensuring good clutch operation. It should be noted that in other embodiments, a plurality of return springs 109 may also be provided. The plurality of return springs 109 are provided around the transmission shaft 106, and are located between the clutch disc 108 and the spring stopper 110.

Referring to FIGS. 1 and 2, a first meshing component is provided on an end face of the clutch disc 108 opposite to the second transmission pulley 107, and a second meshing component is provided on an end face of the second transmission pulley 107 opposite to the clutch disc 108; and when the clutch disc 108 moves between station a and station b along the axial direction of the transmission shaft 106, the first meshing component and the second meshing component form a clutch structure to couple and separate the clutch disc 108 and the second transmission pulley 107. The shapes and designs of the meshing components on the second transmission pulley 107 and the clutch disc 108 are usually selected according to specific transmission systems and requirements to ensure the reliability and stability of the transmission.

Referring to FIGS. 1, 2, 5 and 6, a boss 1082 is provided in the middle of the end face of the clutch disc 108 opposite to the second transmission pulley 107. An annular region around the boss 1082 serves as a second end action region of the clutch lever 105. The first meshing component is several first protruding teeth 1081 arranged at intervals along the circumferential direction of the end face of the boss 1082 opposite to the second transmission pulley 107 and extending toward the second transmission pulley 107. The second meshing component is several second protruding teeth 1071 arranged at intervals along the circumferential direction of the end face of the second transmission pulley 107 opposite to the clutch disc 108 and extending toward the clutch disc 108. The first protruding teeth 1081 and the second protruding teeth 1071 are similar in structure and arranged in opposite directions. Of course, in other embodiments, the first meshing component and the second meshing component may also be other suitable structures such as ratchets and protrusions.

Specifically, the first protruding tooth 1081 and the second protruding tooth 1071 are both designed with helical teeth. Taking the first protruding tooth as an example, an included angle α between a side of the first protruding tooth 1081 and an axial direction is between 5° and 15°, preferably 7° to 10°, such as 7°, 8°, 9° or 10°. The structure of the second protruding tooth 1071 is similar to that of the first protruding tooth 1081, and thus will not be described again. The clutch disc 108 and the second transmission pulley 107 are meshed with helical teeth. Compared with the design of straight tooth meshing, it not only ensures stable and effective transmission, but also eliminates the transmission backlash, while avoiding the risk of the clutch disc 108 not being able to be pulled out due to the straight tooth design. Of course, in other embodiments, the first protruding tooth 1081 and the second protruding tooth 1071 may also be straight teeth.

Referring to FIGS. 1 to 3, the connecting rod mechanism includes a clutch lever 105 and a rotating connecting rod 104. The rotating connecting rod 104 includes a first fulcrum and a first segment and a second segment located on both sides of the first fulcrum. The first fulcrum is located in the middle (the middle here does not refer to a middle position) of the rotating connecting rod 104, and may be mounted on the fixed connecting plate 20, the table body or a fixed component of the table board transmission device through a first mounting bracket 118, so that the first fulcrum of the rotating connecting rod 104 is maintained in a first fixed position during the movement of the clutch pull handle 103. In order to avoid a rotating shaft 112 of the first fulcrum of the rotating connecting rod 104, an avoidance groove 1033 may be provided on the upper surface of the clutch pull handle 103 to prevent the clutch pull handle 103 from interfering with the rotating shaft 112 at the first fulcrum of the rotating connecting rod 104 during the movement. The first segment of the rotating connecting rod 104 is provided with a first waist-shaped hole 1041 along a length direction, and the second segment of the rotating connecting rod 104 is provided with a second waist-shaped hole 1042 along the length direction. Since the first segment and the second segment of the rotating connecting rod 104 are at a certain angle, which may be 180°, or may not be 180°, the length direction of the first waist-shaped hole 1041 and the length direction of the second waist-shaped hole 1042 are also set at a corresponding angle. It can be understood that in other embodiments, the rotating connecting rod 104 may also be of a connecting rod structure of other suitable shapes according to a spatial position.

The clutch pull handle 103 is provided with a first guide pin 111, and a first end of the clutch lever 105 is provided with a second guide pin 113. The first segment of the rotating connecting rod 104 is connected to the clutch pull handle 103 through the first guide pin 111, and the first guide pin 111 is inserted into the first waist-shaped hole 1041 and can slide along the length direction of the first waist-shaped hole 1041. The second segment of the rotating connecting rod 104 is connected to the first end of the clutch lever 105 through the second guide pin 113, and the second guide pin 113 is inserted into the second waist-shaped hole 1042 and can slide along the length direction of the second waist-shaped hole 1042.

The clutch lever 105 has a second fulcrum. The second fulcrum may be fixed to the fixed connecting plate 20, the table body or the fixed component of the table board transmission device through a second mounting bracket 115, so that the second fulcrum of the clutch lever 105 is maintained at a fixed position, thereby forming a lever structure. A second end of the clutch lever 105 is between the clutch disc 108 and the second transmission pulley 107. When the clutch pull handle 103 moves from the first station to the second station, the first guide pin 111 slides along the length direction of the first waist-shaped hole 1041 in the direction away from the first fulcrum, driving the rotating connecting rod 104 to rotate, thereby driving the second guide pin 113 to slide along the length direction of the second waist-shaped hole 1042 in the direction away from the first fulcrum, and further driving the clutch lever 105 to rotate about the second fulcrum. The second end of the clutch lever 105 drives the clutch disc 108 to move in the direction away from the second transmission pulley 107, and contacts the surface of the clutch disc 108 facing the second transmission pulley 107. Under the action of the second end of the clutch lever 105, the clutch disc 108 moves in the direction away from the second transmission pulley 107, so that the clutch disc 108 moves from station a to station b.

It can be understood that the second end of the clutch lever 105 may not be located between the clutch disc 108 and the second transmission pulley 107, but may be located at another position that can move the second end of the clutch lever 105 in the direction away from the second transmission pulley 107, thereby driving the clutch disc 108 to move in the direction away from the second transmission pulley 107.

Referring to FIGS. 1 to 4, the distance between the first end of the clutch lever 105 and a middle fulcrum is greater than the distance between the second end and the middle fulcrum, thereby forming a force-saving lever structure, so that only a small force is required to achieve the clutch between the clutch disc 108 and the second transmission pulley 107 by pushing and pulling the clutch pull handle 103.

Referring to FIGS. 1 to 4, the clutch release device further includes a locking component. The locking component is arranged between the clutch pull handle 103 and the push-pull rail. The locking component includes a buckle 114 and a slot 1021 that cooperate with each other. The buckle 114 is suspended on one of the clutch pull handle 103 and the push-pull rail, and the slot 1021 is arranged on the other of the clutch pull handle 103 and the push-pull rail. The locking component is used to achieve locking when the push-pull rail is in the first station or the second station.

Specifically, the locking component includes, for example, four buckles 114 and four slots 1021, wherein the two buckles 114 on the left are suspended at both ends of the left side of the clutch pull handle 103, respectively, and the two buckles 114 on the right are suspended at both ends of the right side of the clutch pull handle 103, respectively. Correspondingly, both ends of the left push-pull rail 101 are provided with slots 1021 that cooperate with the two buckles 114 on the left side of the clutch pull handle 103, respectively, and both ends of the right push-pull rail 102 are provided with slots 1021 that cooperate with the two buckles 114 on the right side of the clutch pull handle 103, respectively. When the clutch pull handle 103 moves to the first station, the buckles 114 on the left and right sides of the rear end of the clutch pull handle 103 are snapped into the slots 1021 at the rear ends of the left push-pull rail 101 and the right push-pull rail 102 to lock the clutch pull handle 103, respectively. When the clutch pull handle 103 is in the second station, the buckles 114 on the left and right sides of the front end of the clutch pull handle 103 are snapped into the slots 1021 at the front ends of the left push-pull rail 101 and the right push-pull rail 102 to lock the clutch pull handle 103, respectively. The buckle 114 and the clutch pull handle 103 are connected by an arc-shaped transition section 1141. The arc-shaped transition section 1141 can avoid stress concentration of the buckle 114 during deformation, thereby increasing structural strength. Of course, the positions of the slot 1021 and the buckle 114 may be interchanged, that is, the buckle 114 is provided on the push-pull rail, and the slot 1021 is provided on the side of the clutch pull handle 103. In this case, the buckle 114 is connected to the push-pull rail by an arc transition section. The buckle 114 is, for example, a plastic buckle or another buckle with good elasticity, and the elastic principle of the plastic buckle and the connecting rod mechanism is used to achieve the locking of the clutch release device.

In addition, as shown in FIGS. 1 to 4, since the buckle 114 is arranged in a suspended manner, in order to increase the strength of the buckle 114, a reinforcing rib 116 is also provided on the buckle 114 in accordance with its shape. The reinforcing rib 116 is provided on the upper surface of the buckle 114 on the side away from the push-pull rail. The strength of the buckle 114 can be increased by the reinforcing rib 116.

The working principle of the clutch release device is as follows:
When the clutch pull handle 103 is in the first station and the clutch disc 108 is in station a, the clutch pull handle 103 is pulled to move the clutch pull handle 103 from the first station to the second station, triggering the rotation of the rotating connecting rod 104, thereby driving the clutch lever 105 to rotate. The second end of the clutch lever 105 acts on the clutch disc 108, so that the clutch disc 108 overcomes the elastic force of the return spring 109 to move from station a to station b, realizing the separation of the second transmission pulley 107 and the clutch disc 108, thereby separating the table board transmission device from the motor. When resetting is required, the clutch pull handle 103 is pushed to move the clutch pull handle 103 from the second station to the first station, triggering the rotation of the rotating connecting rod 104. Under the action of the return spring 109, the clutch lever 105 is triggered to reset, and under the action of the return spring 109, the clutch disc 108 moves from station b to station a, realizing the coupling of the second transmission pulley 107 and the clutch disc 108, thereby coupling the table board transmission device with the motor. In the whole process, locking is achieved by relying on the locking components between the clutch pull handle 103 and the left push-pull rail 101 and the right push-pull rail 102.

It should be noted that, in some embodiments, the connecting rod mechanism may not be provided with a rotating connecting rod 104, but only with a clutch lever 105, and the clutch lever 105 is provided in a fulcrum-free manner. At this time, multiple positions such as the left sides, right sides, rear sides, upper sides or lower sides of the clutch pull handle 103 and the push-pull rail only need to satisfy that the first end of the clutch lever 105 is fixedly connected to the clutch pull handle 103, and the second end of the clutch lever 105 serves as a free end. When the clutch pull handle 103 moves linearly forward and backward along the push-pull rail, it also moves linearly forward and backward along the transmission shaft 106 synchronously, thereby realizing the coupling and separation of the clutch disc 108 and the second transmission pulley 107.

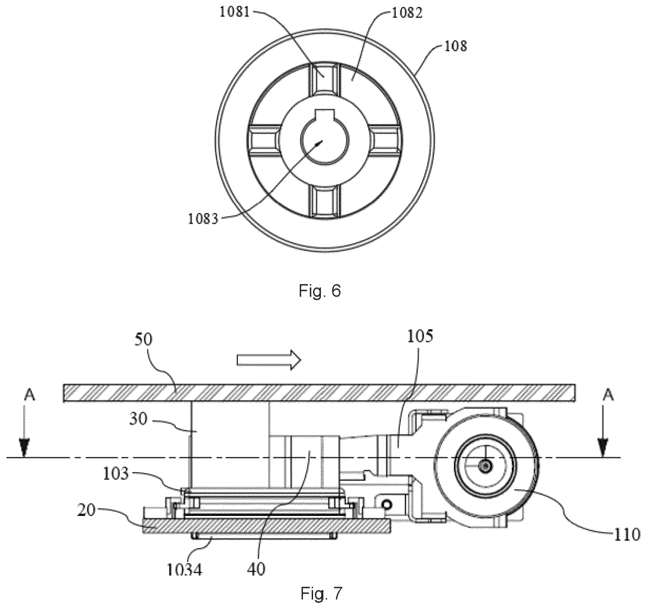

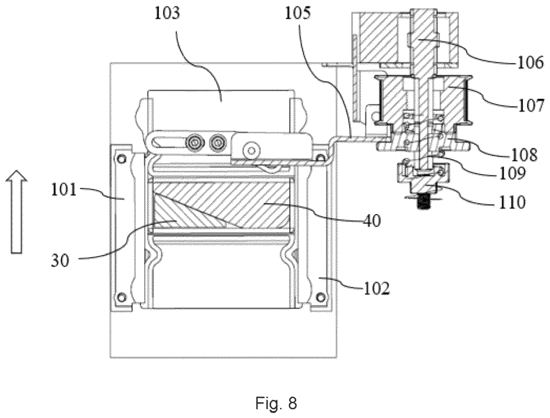

Referring to FIGS. 7 and 8, the clutch release device may also be provided with a self-resetting mechanism. The self-resetting mechanism can realize the automatic reset of the clutch pull handle 103 when the table board 50 (or another external device) is reset in the direction indicated by the arrow on the top of FIG. 7. Specifically, the self-resetting mechanism includes a first resetting component 40 and a second resetting component 30 having ramp surfaces, and the second resetting component 30 is located behind the first resetting component 40. The ramp surfaces of the first resetting component 40 and the second resetting component 30 are placed opposite to each other, the first resetting component 40 is fixed on the clutch pull handle 103, and the second resetting component 30 is fixedly connected to the table board 50. During the reset process of the table board 50, the second resetting component 30 is driven to move, and the movement of the second resetting component 30 triggers the first resetting component 40 to move forward, thereby driving the clutch pull handle 103 to move forward, namely, moving in the direction indicated by the arrow on the left side of FIG. 8 to return to the first station.

It should be noted that since CT medical tables or MR medical tables involve strong magnetic fields and radio frequency signals, the use of magnetic materials around these devices may interfere with or damage the performance of the devices. Therefore, when the clutch release device is applied to a CT medical table or an MR medical table, various components in the clutch release device must be made of non-magnetic materials that can meet the structural strength, such as aluminum alloy, plastic, etc. Of course, in other application scenarios, the clutch release device may also be made of magnetic materials.

In summary, the clutch release device of the present application includes: a fixed connecting plate; a push-pull rail provided on the fixed connecting plate; a clutch pull handle mounted outside the push-pull rail, the clutch pull handle being movable between a first station and a second station along the push-pull rail; a transmission pulley sleeved on a transmission shaft located on one side of the fixed connecting plate and being rotatable about the transmission shaft, the transmission pulley receiving power and torque from the motor to rotate about the transmission shaft; a clutch disc sleeved outside the transmission shaft and being located on one side of the transmission pulley, the clutch disc rotating synchronously with the transmission shaft, and the clutch disc being movable between station a and station b along an axial direction of the transmission shaft; and a connecting rod mechanism provided between the clutch disc and the clutch pull handle, the connecting rod structure being configured such that when the clutch pull handle is in the first station, the clutch disc is in station a, and the clutch disc is coupled to the transmission pulley, and when the clutch pull handle is in the second station, the clutch disc is in station b, and the clutch disc is separated from the transmission pulley. The clutch release device of the present application can be used in a medical table to implement the transmission state control between the motor and the table board in the medical table, which can ensure that the table board of the medical table can be safely moved in various situations, and reduce the risk of injury to patients.

In the clutch release device of the present application, the working direction of the clutch handle is set to be parallel to the axial direction of the transmission shaft, so that the working direction of the clutch handle is consistent with the clutch direction, which is convenient for the operation of the clutch.

In the clutch release device of the present application, helical tooth meshing is adopted between the clutch disc and the transmission pulley. Compared with the design of straight tooth meshing, it not only ensures stable and effective transmission, but also eliminates the transmission backlash, while avoiding the risk of the clutch disc not being able to be pulled out due to the straight tooth design.

The clutch lever of the clutch release device of the present application adopts a labor-saving lever structure. During the operation, only a small force is required to realize the clutch between the clutch disc and the transmission pulley.

In the clutch release device of the present application, a buckle and a slot are adopted to realize clutch locking, which is ingeniously designed, flexible and reliable.

It is obvious to those skilled in the art that the present application is not limited to the details of the above exemplary embodiments, and that the present application can be implemented in other specific forms without departing from the essential features of the present application as defined in the attached set of claims.

The above embodiments are only used to describe the technical solutions of the present application without limiting it. Although the present application has been described in detail with reference to the preferred embodiments, those of ordinary skill in the art should understand that the technical solutions of the present application may be modified without departing from the scope of the technical solutions of the present application as defined in the attached set of claims.

## Claims

1. A clutch release device, comprising:
a push-pull rail (101, 102);
a clutch pull handle (103) mounted on the push-pull rail (101, 102), the clutch pull handle (103) being moveable between a first station and a second station along the push-pull rail (101, 102);
a transmission wheel sleeved outside the transmission shaft (106) located at one side of the push-pull rail (101, 102) and being rotatable about the transmission shaft (106), the transmission wheel receiving power and torque from a motor to rotate about the transmission shaft (106);
a clutch disc (108) sleeved outside the transmission shaft (106) and located on one side of the transmission wheel, the clutch disc (108) rotating synchronously with the transmission shaft (106), and the clutch disc (108) being movable between station a and station b along an axial direction of the transmission shaft (106); and
a connecting rod mechanism provided between the clutch disc (108) and the clutch pull handle (103), the connecting rod mechanism being configured such that when the clutch pull handle (103) is in the first station, the clutch disc (108) is in station a, and the clutch disc (108) is coupled to and rotates synchronously with the transmission wheel, and when the clutch pull handle (103) is in the second station, the clutch disc (108) is in station b, and the clutch disc (108) is separated from the transmission wheel,
**characterized in that** a working direction of the clutch pull handle (103) is parallel to the axial direction of the transmission shaft (106).

2. The clutch release device according to claim 1, further comprising a locking component provided between the clutch pull handle (103) and the push-pull rail (101, 102), the locking component being used to achieve locking when the push-pull rail (101, 102) is in the first station or the second station.

3. The clutch release device according to claim 2, **characterized in that** the locking component comprises a buckle (114) and a slot (1021) that cooperate with each other, the buckle (114) is suspended on one of the clutch pull handle (103) and the push-pull rail (101, 102), and the slot (1021) is provided on the other of the clutch pull handle (103) and the push-pull rail (101, 102).

4. The clutch release device according to claim 3, **characterized in that** the buckle (114) is connected to the clutch pull handle (103) or the push-pull rail (101, 102) by an arc-shaped transition section (1141).

5. The clutch release device according to claim 1, **characterized in that** the clutch release device comprises two push-pull rails (101, 102) provided in parallel, which are provided on both sides of the clutch pull handle (103), respectively.

6. The clutch release device according to claim 5, **characterized in that** at least two guide portions (1031) are spaced apart on both sides of the clutch pull handle (103), respectively, and the guide portions (1031) are located between the clutch pull handle (103) and the push-pull rail (101, 102) on the same side.

7. The clutch release device according to claim 1, **characterized in that** the connecting rod mechanism comprises a clutch lever (105) and a rotating connecting rod (104);
the rotating connecting rod (104) comprises a first fulcrum and a first segment and a second segment located on both sides of the fulcrum, the first fulcrum is rotatably mounted at a first fixed position, the first segment is connected to the clutch pull handle (103) through a first guide pin (111) provided on the clutch pull handle (103), the first guide pin (111) can slide along a length direction of the first segment, the second segment is connected to the clutch lever (105) through a second guide pin (113) provided on a first end of the clutch lever (105), the second guide pin (113) can slide along a length direction of the second segment of the rotating connecting rod (104);
a second fulcrum of the clutch lever (105) is fixed at a second fixed position, and the positions of the first fixed position and the second fixed position remain relatively unchanged; and
when the clutch pull handle (103) moves from the first station to the second station, the first guide pin (111) slides along the length direction of the first segment in a direction away from the first fulcrum, driving the rotating connecting rod (104) to rotate, thereby driving the second guide pin (113) to slide along the length direction of the second segment in the direction away from the first fulcrum, and further driving the clutch lever (105) to rotate about the second fulcrum, driving the clutch disc (108) to move in a direction away from the transmission wheel, and moving the clutch disc (108) from station a to station b.

8. The clutch release device according to claim 7, **characterized in that** a distance between the first end of the clutch lever (105) and the second fulcrum is greater than a distance between a second end and the second fulcrum.

9. The clutch release device according to claim 1, further comprising a return spring (109), the return spring (109) being used to return the clutch disc (108) to station a after the clutch disc (108) is released.

10. The clutch release device according to claim 1, **characterized in that** a first meshing component is provided on an end face of the clutch disc (108) opposite to the transmission wheel, and a second meshing component is provided on an end face of the transmission wheel opposite to the clutch disc (108); and when the clutch disc (108) moves between station a and station b along the axial direction of the transmission shaft (106), the first meshing component and the second meshing component form a clutch structure to couple and separate the clutch disc (108) and the transmission wheel.

11. The clutch release device according to claim 10, **characterized in that** the first meshing component and the second meshing component are designed with helical teeth (1071, 1081), and an included angle between a side of a helical tooth (1071, 1081) and an axial direction of the helical tooth (1071, 1081) is between 5° and 15°.

12. The clutch release device according to claim 11, **characterized in that** the included angle between the side of the helical tooth (1071, 1081) and the axial direction of the helical tooth (1071, 1081) is between 7° and 10°.

13. The clutch release device according to claim 1, further comprising a self-resetting mechanism, comprising a first resetting component (40) and a second resetting component (30) having ramp surfaces;
wherein the ramp surfaces of the first resetting component (40) and the second resetting component (30) are provided opposite to each other, the first resetting component (40) is fixed to the clutch pull handle (103), and the second resetting component (30) is fixedly connected to an external device; and
in a resetting process of the external device, the second resetting component (30) is driven to move, and the movement of the second resetting component (30) triggers the movement of the first resetting component (40), thereby driving the clutch pull handle (103) to move to return to the first station.

14. A medical table, comprising:
a table body;
a table board (50) provided on the table body and being movable relative to the table body;
a table board transmission device (117) provided on the table body, the table board transmission device (117) being connected to the table board (50) and being used to drive the table board (50) to move, and the table board transmission device (117) having a transmission shaft (106);
a motor provided on the table body; and
the clutch release device of any one of claims 1 - 13, wherein the clutch release device is provided between the motor and the table board transmission device (117), and is used to control a transmission state between the table board transmission device (117) and the motor, and wherein the push-pull rail (101, 102) of the clutch release device is provided on the table body.

15. The medical table according to claim 14, **characterized in that** a moving direction of the table board (50) and a working direction of the clutch pull handle (103) are perpendicular to each other.

## Patentansprüche

1. Kupplungslösevorrichtung, die Folgendes umfasst:
eine Druck-Zug-Schiene (101, 102);
einen Kupplungszuggriff (103), der an der Druck-Zug-Schiene (101, 102) montiert ist, wobei der Kupplungszuggriff (103) zwischen einer ersten Station und einer zweiten Station entlang der Druck-Zug-Schiene (101, 102) beweglich ist;
ein Übertragungsrad, das außerhalb der Übertragungswelle (106) aufgeschoben ist, die sich auf einer Seite der Druck-Zug-Schiene (101, 102) befindet und um die Übertragungswelle (106) drehbar ist, wobei das Übertragungsrad Leistung und Drehmoment von einem Motor erhält, um sich um die Übertragungswelle (106) zu drehen;
eine außerhalb der Übertragungswelle (106) aufgeschobene Kupplungsscheibe (108), die sich auf einer Seite des Übertragungsrads befindet, wobei sich die Kupplungsscheibe (108) mit der Übertragungswelle (106) synchron dreht und die Kupplungsscheibe (108) zwischen Station a und Station b entlang einer Axialrichtung der Übertragungswelle (106) beweglich ist; und
ein zwischen der Kupplungsscheibe (108) und dem Kupplungszuggriff (103) vorgesehener Verbindungsstangenmechanismus, wobei der Verbindungsstangenmechanismus so ausgelegt ist, dass, wenn sich der Kupplungszuggriff (103) in der ersten Station befindet, sich die Kupplungsscheibe (108) in Station a befindet und die Kupplungsscheibe (108) mit dem Übertragungsrad gekoppelt ist und sich mit diesem synchron dreht, und wenn sich der Kupplungszuggriff (103) in der zweiten Station befindet, sich die Kupplungsscheibe (108) in Station b befindet und die Kupplungsscheibe (108) vom Übertragungsrad getrennt ist,
**dadurch gekennzeichnet, dass** eine Arbeitsrichtung des Kupplungszuggriffs (103) parallel zur Axialrichtung der Übertragungswelle (106) ist.

2. Kupplungslösevorrichtung nach Anspruch 1, ferner umfassend eine Verriegelungskomponente, die zwischen dem Kupplungszuggriff (103) und der Druck-Zug-Schiene (101, 102) vorgesehen ist, wobei die Verriegelungskomponente verwendet wird, um eine Verriegelung zu erreichen, wenn sich die Druck-Zug-Schiene (101, 102) in der ersten Station oder der zweiten Station befindet.

3. Kupplungslösevorrichtung nach Anspruch 2, **dadurch gekennzeichnet, dass** die Verriegelungskomponente eine Schnalle (114) und einen Schlitz (1021) umfasst, die miteinander zusammenwirken, wobei die Schnalle (114) am Kupplungszuggriff (103) und/oder an der Druck-Zug-Schiene (101, 102) aufgehängt ist und der Schlitz (1021) an dem anderen des Kupplungszuggriffs (103) und der Druck-Zug-Schiene (101, 102) vorgesehen ist.

4. Kupplungslösevorrichtung nach Anspruch 3, **dadurch gekennzeichnet, dass** die Schnalle (114) über einen bogenförmigen Übergangsabschnitt (1141) mit dem Kupplungszuggriff (103) oder der Druck-Zug-Schiene (101, 102) verbunden ist.

5. Kupplungslösevorrichtung nach Anspruch 1, **dadurch gekennzeichnet, dass** die Kupplungslösevorrichtung zwei parallel vorgesehene Druck-Zug-Schienen (101, 102) umfasst, die auf beiden Seiten des Kupplungszuggriffs (103) vorgesehen sind.

6. Kupplungslösevorrichtung nach Anspruch 5, **dadurch gekennzeichnet, dass** auf beiden Seiten des Kupplungszuggriffs (103) jeweils mindestens zwei Führungsabschnitte (1031) beabstandet sind und sich die Führungsabschnitte (1031) zwischen dem Kupplungszuggriff (103) und der Druck-Zug-Schiene (101, 102) auf derselben Seite befinden.

7. Kupplungslösevorrichtung nach Anspruch 1, **dadurch gekennzeichnet, dass** der Verbindungsstangenmechanismus einen Kupplungshebel (105) und eine sich drehende Verbindungsstange (104) umfasst;
wobei die sich drehende Verbindungsstange (104) einen ersten Drehpunkt und ein erstes Segment und ein zweites Segment, die sich auf beiden Seiten des Drehpunkts befinden, umfasst, wobei der erste Drehpunkt in einer ersten festen Position drehbar montiert ist, wobei das erste Segment über einen am Kupplungszuggriff (103) vorgesehenen ersten Führungsstift (111) mit dem Kupplungszuggriff (103) verbunden ist, wobei der erste Führungsstift (111) entlang einer Längsrichtung des ersten Segments verschiebbar ist, wobei das zweite Segment über einen an einem ersten Ende des Kupplungshebels (105) vorgesehenen zweiten Führungsstift (113) mit dem Kupplungshebel (105) verbunden ist, wobei der zweite Führungsstift (113) entlang einer Längsrichtung des zweiten Segments der sich drehenden Verbindungsstange (104) verschiebbar ist;
wobei ein zweiter Drehpunkt des Kupplungshebels (105) in einer zweiten festen Position befestigt ist und die Positionen der ersten festen Position und der zweiten festen Position relativ unverändert bleiben und
wobei, wenn sich der Kupplungszuggriff (103) von der ersten Station zur zweiten Station bewegt, sich der erste Führungsstift (111) entlang der Längsrichtung des ersten Segments in eine Richtung vom ersten Drehpunkt weg verschiebt, wodurch die sich drehende Verbindungsstange (104) zum Drehen angetrieben wird, wodurch der zweite Führungsstift (113) zum Verschieben entlang der Längsrichtung des zweiten Segments in Richtung vom ersten Drehpunkt weg angetrieben wird, und ferner der Kupplungshebel (105) angetrieben wird, sich um den zweiten Drehpunkt zu drehen, wodurch die Kupplungsscheibe (108) angetrieben wird, sich in eine Richtung vom Übertragungsrad weg zu bewegen, und wodurch die Kupplungsscheibe (108) von Station a zu Station b bewegt wird.

8. Kupplungslösevorrichtung nach Anspruch 7, **dadurch gekennzeichnet, dass** der Abstand zwischen dem ersten Ende des Kupplungshebels (105) und dem zweiten Drehpunkt größer ist als der Abstand zwischen dem zweiten Ende und dem zweiten Drehpunkt.

9. Kupplungslösevorrichtung nach Anspruch 1, ferner umfassend eine Rückstellfeder (109), wobei die Rückstellfeder (109) dazu verwendet wird, die Kupplungsscheibe (108) nach dem Lösen der Kupplungsscheibe (108) zu Station a zurückzubringen.

10. Kupplungslösevorrichtung nach Anspruch 1, **dadurch gekennzeichnet, dass** an einer dem Übertragungsrad gegenüberliegenden Stirnseite der Kupplungsscheibe (108) eine erste Eingriffskomponente und an einer der Kupplungsscheibe (108) gegenüberliegenden Stirnseite des Übertragungsrads eine zweite Eingriffskomponente vorgesehen ist; und wenn sich die Kupplungsscheibe (108) zwischen Station a und Station b entlang der Axialrichtung der Übertragungswelle (106) bewegt, die erste Eingriffskomponente und die zweite Eingriffskomponente eine Kupplungsstruktur zum Koppeln und Trennen der Kupplungsscheibe (108) und des Übertragungsrads bilden.

11. Kupplungslösevorrichtung nach Anspruch 10, **dadurch gekennzeichnet, dass** die erste Eingriffskomponente und die zweite Eingriffskomponente mit einer Schrägverzahnung (1071, 1081) ausgestaltet sind und ein eingeschlossener Winkel zwischen einer Seite eines Schrägzahns (1071, 1081) und einer Axialrichtung des Schrägzahns (1071, 1081) zwischen 5° und 15° beträgt.

12. Kupplungslösevorrichtung nach Anspruch 11, **dadurch gekennzeichnet, dass** der eingeschlossene Winkel zwischen der Seite des Schrägzahns (1071, 1081) und der Axialrichtung des Schrägzahns (1071, 1081) zwischen 7° und 10° beträgt.

13. Kupplungslösevorrichtung nach Anspruch 1, die ferner einen Selbstrücksetzmechanismus umfasst, der eine erste Rücksetzkomponente (40) und eine zweite Rücksetzkomponente (30) mit Rampenflächen umfasst;
wobei die Rampenflächen der ersten Rücksetzkomponente (40) und der zweiten Rücksetzkomponente (30) einander gegenüberliegend vorgesehen sind, wobei die erste Rücksetzkomponente (40) am Kupplungszuggriff (103) befestigt ist und die zweite Rücksetzkomponente (30) mit einer externen Vorrichtung fest verbunden ist; und
wobei in einem Rücksetzvorgang der externen Vorrichtung die zweite Rücksetzkomponente (30) angetrieben wird, sich zu bewegen, und die Bewegung der zweiten Rücksetzkomponente (30) die Bewegung der ersten Rücksetzkomponente (40) auslöst, wodurch der Kupplungszuggriff (103) angetrieben wird, sich zu bewegen, um zur ersten Station zurückzukehren.

14. Medizinischer Tisch, umfassend:
einen Tischkörper;
eine Tischplatte (50), die auf dem Tischkörper vorgesehen und relativ zum Tischkörper beweglich ist;
eine Tischplattenübertragungsvorrichtung (117), die am Tischkörper vorgesehen ist, wobei die Tischplattenübertragungsvorrichtung (117) mit der Tischplatte (50) verbunden ist und dazu verwendet wird, die Tischplatte (50) anzutreiben, sich zu bewegen, und wobei die Tischplattenübertragungsvorrichtung (117) eine Übertragungswelle (106) aufweist;
einen Motor, der am Tischkörper vorgesehen ist; und
die Kupplungslösevorrichtung nach einem der Ansprüche 1-13, wobei die Kupplungslösevorrichtung zwischen dem Motor und der Tischplattenübertragungsvorrichtung (117) vorgesehen ist und verwendet wird, um einen Übertragungszustand zwischen der Tischplattenübertragungsvorrichtung (117) und dem Motor zu steuern, und wobei die Druck-Zug-Schiene (101, 102) der Kupplungslösevorrichtung am Tischkörper vorgesehen ist.

15. Medizinischer Tisch nach Anspruch 14, **dadurch gekennzeichnet, dass** die Bewegungsrichtung der Tischplatte (50) und die Arbeitsrichtung des Kupplungszuggriffs (103) senkrecht zueinander stehen.

## Revendications

1. Dispositif de libération d'embrayage, comprenant :
un rail de poussée-traction (101, 102) ;
une poignée de traction d'embrayage (103) montée sur le rail de poussée-traction (101, 102), la poignée de traction d'embrayage (103) étant mobile entre une première station et une seconde station le long du rail de poussée-traction (101, 102) ;
une roue de transmission emmanchée à l'extérieur de l'arbre de transmission (106) située d'un côté du rail de poussée-traction (101, 102) et pouvant tourner autour de l'arbre de transmission (106), la roue de transmission recevant la puissance et le couple d'un moteur pour tourner autour de l'arbre de transmission (106) ;
un disque d'embrayage (108) emmanché à l'extérieur de l'arbre de transmission (106) et situé sur un côté de la roue de transmission, le disque d'embrayage (108) tournant en synchronisme avec l'arbre de transmission (106), et le disque d'embrayage (108) étant mobile entre la station a et la station b le long d'une direction axiale de l'arbre de transmission (106) ; et
un mécanisme de bielle prévu entre le disque d'embrayage (108) et la poignée de traction d'embrayage (103), le mécanisme de bielle étant configuré de telle sorte que, lorsque la poignée de traction d'embrayage (103) est dans la première station, le disque d'embrayage (108) est dans la station a, et le disque d'embrayage (108) est couplé à la roue de transmission et tourne de manière synchrone avec celle-ci, et lorsque la poignée de traction d'embrayage (103) est dans la seconde station, le disque d'embrayage (108) est dans la station b, et le disque d'embrayage (108) est séparé de la roue de transmission,
**caractérisé en ce qu'**une direction de travail de la poignée de traction d'embrayage (103) est parallèle à la direction axiale de l'arbre de transmission (106).

2. Dispositif de libération d'embrayage selon la revendication 1, comprenant en outre un composant de verrouillage prévu entre la poignée de traction d'embrayage (103) et le rail de poussée-traction (101, 102), le composant de verrouillage étant utilisé pour réaliser un verrouillage lorsque le rail de poussée-traction (101, 102) est dans la première station ou la seconde station.

3. Dispositif de libération d'embrayage selon la revendication 2, **caractérisé en ce que** le composant de verrouillage comprend une boucle (114) et une fente (1021) qui coopèrent l'une avec l'autre, la boucle (114) est suspendue à l'un de la poignée de traction d'embrayage (103) et du rail de poussée-traction (101, 102), et la fente (1021) est prévue sur l'autre de la poignée de traction d'embrayage (103) et du rail de poussée-traction (101, 102).

4. Dispositif de libération d'embrayage selon la revendication 3, **caractérisé en ce que** la boucle (114) est reliée à la poignée de traction d'embrayage (103) ou au rail de poussée-traction (101, 102) par une section de transition en forme d'arc (1141).

5. Dispositif de libération d'embrayage selon la revendication 1, **caractérisé en ce que** le dispositif de libération d'embrayage comporte deux rails de poussée-traction (101, 102) prévus en parallèle, qui sont prévus respectivement des deux côtés de la poignée de traction d'embrayage (103).

6. Dispositif de libération d'embrayage selon la revendication 5, **caractérisé en ce qu'**au moins deux parties de guidage (1031) sont espacées respectivement des deux côtés de la poignée de traction d'embrayage (103), et les parties de guidage (1031) sont situées entre la poignée de traction d'embrayage (103) et le rail de poussée-traction (101, 102) du même côté.

7. Dispositif de libération d'embrayage selon la revendication 1, **caractérisé en ce que** le mécanisme de bielle comprend un levier d'embrayage (105) et une bielle rotative (104) ;
la bielle rotative (104) comprend un premier point d'appui et un premier segment et un second segment situés des deux côtés du point d'appui, le premier point d'appui est monté de manière rotative à une première position fixe, le premier segment est relié à la poignée de traction d'embrayage (103) par l'intermédiaire d'une première broche de guidage (111) prévue sur la poignée de traction d'embrayage (103), la première broche de guidage (111) peut coulisser le long d'une direction longitudinale du premier segment, le second segment est relié au levier d'embrayage (105) par l'intermédiaire d'une seconde broche de guidage (113) prévue sur une première extrémité du levier d'embrayage (105), la seconde broche de guidage (113) peut coulisser le long d'une direction longitudinale du second segment de la bielle rotative (104) ;
un second point d'appui du levier d'embrayage (105) est fixé à une seconde position fixe, et les positions de la première position fixe et de la seconde position fixe restent relativement inchangées ; et
lorsque la poignée de traction d'embrayage (103) se déplace de la première station à la seconde station, la première broche de guidage (111) coulisse le long de la direction longitudinale du premier segment dans une direction s'éloignant du premier point d'appui, entraînant la bielle rotative (104) en rotation, entraînant ainsi la seconde broche de guidage (113) à coulisser le long de la direction longitudinale du second segment dans la direction s'éloignant du premier point d'appui, et entraînant en outre le levier d'embrayage (105) à tourner autour du second point d'appui, entraînant le disque d'embrayage (108) à se déplacer dans une direction s'éloignant de la roue de transmission, et déplaçant le disque d'embrayage (108) de la station a à la station b.

8. Dispositif de libération d'embrayage selon la revendication 7, **caractérisé en ce qu'**une distance entre la première extrémité du levier d'embrayage (105) et le second point d'appui est supérieure à une distance entre une seconde extrémité et le second point d'appui.

9. Dispositif de libération d'embrayage selon la revendication 1, comprenant en outre un ressort de rappel (109), le ressort de rappel (109) étant utilisé pour ramener le disque d'embrayage (108) à la station a après la libération du disque d'embrayage (108).

10. Dispositif de libération d'embrayage selon la revendication 1, **caractérisé en ce qu'**un premier composant d'engrènement est prévu sur une face d'extrémité du disque d'embrayage (108) opposée à la roue de transmission, et un second composant d'engrènement est prévu sur une face d'extrémité de la roue de transmission opposée au disque d'embrayage (108) ; et lorsque le disque d'embrayage (108) se déplace entre la station a et la station b le long de la direction axiale de l'arbre de transmission (106), le premier composant d'engrènement et le second composant d'engrènement forment une structure d'embrayage pour accoupler et séparer le disque d'embrayage (108) et la roue de transmission.

11. Dispositif de libération d'embrayage selon la revendication 10, **caractérisé en ce que** le premier composant d'engrènement et le second composant d'engrènement sont conçus avec des dents hélicoïdales (1071, 1081), et un angle inclus entre un côté d'une dent hélicoïdale (1071, 1081) et une direction axiale de la dent hélicoïdale (1071, 1081) est compris entre 5° et 15°.

12. Dispositif de libération d'embrayage selon la revendication 11, **caractérisé en ce que** l'angle inclus entre le côté de la dent hélicoïdale (1071, 1081) et la direction axiale de la dent hélicoïdale (1071, 1081) est compris entre 7° et 10°.

13. Dispositif de libération d'embrayage selon la revendication 1, comprenant en outre un mécanisme de rappel automatique, comprenant un premier composant de rappel (40) et un second composant de rappel (30) ayant des surfaces de rampe ;
les surfaces de rampe du premier composant de rappel (40) et du second composant de rappel (30) étant disposées l'une en face de l'autre, le premier composant de rappel (40) étant fixé à la poignée de traction d'embrayage (103), et le second composant de rappel (30) étant relié de manière fixe à un dispositif externe ; et
dans un processus de rappel du dispositif externe, le second composant de rappel (30) étant entraîné en déplacement, et le mouvement du second composant de rappel (30) déclenchant le mouvement du premier composant de rappel (40), entraînant ainsi le déplacement de la poignée de traction d'embrayage (103) pour revenir à la première station.

14. Table médicale, comprenant :
un corps de table ;
un plateau de table (50) prévu sur le corps de table et mobile par rapport au corps de table ;
un dispositif de transmission de plateau de table (117) prévu sur le corps de table, le dispositif de transmission de plateau de table (117) étant relié au plateau de table (50) et étant utilisé pour entraîner le déplacement du plateau de table (50), et le dispositif de transmission de plateau de table (117) ayant un arbre de transmission (106) ;
un moteur prévu sur le corps de table ; et
le dispositif de libération d'embrayage selon l'une quelconque des revendications 1 à 13, le dispositif de libération d'embrayage étant prévu entre le moteur et le dispositif de transmission de plateau de table (117), et étant utilisé pour commander un état de transmission entre le dispositif de transmission de plateau de table (117) et le moteur, et le rail de poussée-traction (101, 102) du dispositif de libération d'embrayage étant prévu sur le corps de table.

15. Table médicale selon la revendication 14, **caractérisée en ce qu'**une direction de déplacement du plateau de table (50) et une direction de travail de la poignée de traction d'embrayage (103) sont perpendiculaires l'une à l'autre.
